# EUROPEAN PATENT SPECIFICATION

(11) **EP 2 327 782 B1**
(45) Date of publication and mention of the grant of the patent: **25.02.2015**
(21) Application number: 08800572.3
(22) Date of filing: 04.09.2008
(51) Int. Cl.: C12N 15/11, C12N 15/10, C12N 15/66

(54) **DNA MOLECULE FOR EXPRESSING HARIPIN RNA, THE CONSTRUCTING METHOD AND THE USE THEREOF**
DNA-MOLEKÜL ZUR EXPRESSION VON HAARNADEL-RNA, KONSTRUKTIONSVERFAHREN DAFÜR UND VERWENDUNG DAVON
MOLÉCULE D'ADN POUR L'EXPRESSION D'ARN EN ÉPINGLE À CHEVEUX, SON PROCÉDÉ DE CONSTRUCTION ET SON UTILISATION

(43) Date of publication of application: 01.06.2011
(73) Proprietor: Biotechnology Research Institute Chinese Academy of Agricultural Sciences, Haidian District Beijing 100081 (CN)
(72) Inventor:
(74) Representative: Isarpatent
(86) International application number: PCT/CN2008/001576
(87) International publication number: WO 2010/025584

(56) References cited:
- WO-A2-2004/065582
- WO-A2-2009/120372
- CN-A- 1 710 079
- CN-A- 101 139 615
- US-A1- 2008 021 205
- US-A1- 2008 021 205
- HEINRICH J ET AL: "Linear closed mini DNA generated by the prokaryotic cleaving-joining enzyme TelN is functional in mammalian cells", JOURNAL OF MOLECULAR MEDICINE, SPRINGER VERLAG, DE, vol. 80, no. 10, 1 October 2002 (2002-10-01), pages 648-654, XP002580374, ISSN: 0946-2716, DOI: 10.1007/S00109-002-0362-2 [retrieved on 2002-08-28]
- SEN G, WEHRMAN TS, MYERS JW, BLAU HM: "Restriction enzyme-generated siRNA (REGS) vectors and libraries", NAT GENET, vol. 36, no. 2, 4 January 2004 (2004-01-04), pages 183-189, XP002326034,
- PAVLINA KONSTANTINOVA ET AL.: 'Trans-inhibition of HIV-1 by a long hairpin RNA expressed within the viral genome' RETROVIROLOGY vol. 4, no. 15, 01 March 2007, pages 1 - 14, XP021025293
- LEI WANG ET AL.: 'Rolling circle amplification-mediated hairpin RNA (RMHR) library construction in plants' NUCLEIC ACIDS RESEARCH vol. 36, no. 22, 31 October 2008, page E149, XP008144083

## Description

### FIELD OF THE INVENTION

The invention describes a DNA molecule for expressing hairpin RNA, and relates to a constructing method of a DNA molecule for expressing hairpin RNA and use thereof.

### BACKGROUD OF THE INVENTION

RNA-mediated gene silencing/RNA interfering (RNAi) can induce the degradation of the specific homologous gene mRNA, is an effective tool for inhibiting the gene expression in eukaryotes and is widely used in the identification of gene function and the genetic engineering. By using hairpin RNA (hpRNA), the gene silencing efficiency can generally reach over 70%, which is much higher than that obtained with the antisense RNA strategy. Therefore, hpRNA is the most widely used technology for gene silencing in animals, plants and microorganisms (Wesley, S. V., Plant J. (2001) 27, 581-590).

There are a variety of methods for constructing hpRNA at present, the most commonly used is two-step method, i.e. a target sequence is inserted into a vector, then the sequence is inversely inserted once again to form a inverted repeat structure, which can transcribe the hpRNA. The target sequences are inserted into a vector in a reverse orientation using the recombinase by twice recombination in the Gateway recombinant technology (Tomari, Y. Genes Dev (2005) 19, 517-529). Recently artificially synthesized miRNA (micro RNA) also can be specifically silencing target genes (Miki, D. Plant Cell Physiol (2004) 45, 490-495). However, these methods can only manipulate one DNA fragment at a time, which is suitable for constructing one or a few target gene silencing vectors, is time ineffective and hard sledding, and is unable to construct the gene silencing library.

In recent years, the short hairpin RNA (shRNA) library is constructed by short DNA fragment in animals. Luo et. al. constructed shRNA expression library using SPEED (siRNA production by enzymatic engineering of DNA) strategy (Luo, B. Proc Natl Acad Sci USA, (2004) 101, 5494-5499). Shirane et. al. also constructed shRNA expression library using REGS (restriction enzyme-generated siRNA) system (Shirane, D., Nat Genet (2004) 36, 190-196). Sen et. al. constructed shRNA expression library utilizing EPRIL (enzymatic production of RNAi library) technology (Sen, G., Wehrman, Nat Genet (2004) 36, 183-189). However, these technologies adopt similar methods, they all utilize the property of the restriction endonuclease MmeI, and the lengths of the stems in the siRNA library or the shRNA library constructed are all about 20bp. This short hairpin structure RNA (shRNA) is not only low silencing efficiency, but also poor silencing effect compared to the long hairpin RNA (lhRNA) with long stems (such as above 40bp).Thus how to construct a hairpin RNA (lhRNA) library with long stems is a challenge at present. There is no any report about the construction of a hairpin RNA library with the stem length above 40bp.

US 2008/021205 A1 discloses methods and compositions for producing hRNA, wherein in the methods an initial nucleic acid corresponding to the target nucleic acid of interest is converted to an intermediate nucleic acid, which then, following an optional size modification step, is converted to a linear dsDNA that includes at least one copy of the hRNA expression module of interest, or a precursor thereof.

A hairpin structure and its use are described in WO 2004/065582 A2, wherein the hairpin nucleic acid structure can be used in a method of amplification of a template nucleic acid sequence that substantially reduces polymers-induced errors.

In addition, Heinrich, J. et al., "Linear closed mini DNA generated by the prokaryotic cleaving-joining enzyme TelN is functional in mammalian cells", J. Mol. Med. (2002), 80, 648-654 describe the prokaryotic telomerase protelomerase TelN of bacteriophage N15 with cleaving-joining activity, which converts circular plasmid DNA into linear covalently closed dumbbell-shaped molecules in a single-step enzyme reaction when acting on a telomere resolution site *telRL*.

### DESCRIPTION OF THE INVENTION

Described is a DNA molecule for expressing hairpin RNA, a constructing method and use thereof.

The DNA molecule for expressing hairpin is a closed single-strand cyclic molecule consisting of four single-strand DNA fragments A, B, C and D; and said four fragments A, B, C and D are linked in the order of 1) or 2) as follows:
1) A-C-B-D;
2) A-D-B-C;
   A is a fragment selected from a sense or a antisense strand of the target double-strand DNA fragment, and the size of said A is 40-3000nt;
   B is a fragment which is reversely complementary to A;
   C and D are DNA fragments with the stem-loop structure, and said C and D fragments meet the requirement of 3) or 4) as follows:
3) The nucleotide sequence of said fragment C or D comprises at least one sequence e, said sequence e forms a recognition sequence R of restriction endonuclease with the complementary sequence thereof;
4) The nucleotide sequence of said fragment C comprises at least one sequence e, said sequence e forms a recognition sequence R of restriction endonuclease with the complementary sequence thereof; the nucleotide sequence of said fragment D comprises at least one sequence f, said sequence f forms a recognition sequence S of restriction endonuclease with the complementary sequence thereof; said recognition sequences R and S are recognized by the different restriction endonucleases.

Wherein, the size of said A also can be 100-2000nt, 100-1000nt, 200-800nt or 200-600nt. The nucleotide sequences of said C and D can be same, such as SEQ ID NO:4 in the sequence list, or different, without any other restriction, for example the sequences are SEQ ID NO:1 and SEQ ID NO:2 in the sequence list respectively. The loop of the stem-loop structure of said C or D also can comprise an intron sequence or not, the length can be designed according to the requirement, it may be long or short. For example, SEQ ID NO:3 in the sequence list provides a stem-loop structure whose loop comprises a intron sequence. The DNA fragments of said C and D may be obtained by using various methods in the art, such as artificial synthesis or single-prime PCR.

An object of the invention is to provide a constructing method for the DNA molecule for expressing the hairpin RNA.

The constructing method for the DNA molecule for expressing the hairpin RNA of the invention comprises linking three O, P and Q DNA fragments into a closed single-strand cyclic molecule.

Said fragment O is a target double-strand DNA fragment with two cohesive ends which can not be self linked.

Said fragments P and Q are DNA fragments with stem-loop structure having cohesive ends respectively, the cohesive ends of said fragments P and Q are matched with the two cohesive ends of said fragment O, and said fragments P and Q meet the requirement of i) or ii) as follows:
i) the nucleotide sequence of fragment P or Q comprises at least one sequence e, said sequence e forms a recognition sequence R of restriction endonuclease with the complemented sequence thereof;
ii) the nucleotide sequence of said fragment P comprises at least one sequence e, said sequence e forms a recognition sequence R of restriction endonuclease with the complemented sequence thereof; the nucleotide sequence of said fragment Q comprises at least one sequence f, said sequence f forms a recognition sequence S of restriction endonuclease with the complemented sequence thereof; said recognition sequence R and S are recognized by the different restriction endonucleases.

Said fragments P and Q are DNA fragments with stem-loop structure having cohesive ends respectively, the cohesive ends of said fragments P and Q are matched with the two cohesive ends of said fragment O, this ensures that the cohesive ends of said fragment P or Q can not be self linked and they can not be linked into P-P, Q-Q and P-Q closed single-strand cyclic DNA in the enzyme-chain reaction.

Wherein, said three DNA fragments O, P and Q may be obtained by using various methods in the art, such as artificially synthesis or cloning, said fragment P and Q of the invention are artificially synthesized. The length of the projecting cohesive end of said fragment O can be above 1nt, it also can be in the range of 1-9nt or 1-20nt, said fragment O can be obtained through PCR amplification or PCR product enzyme cleavage (a specific endonuclease enzyme cleavage site is introduced into PCR product) or linking the specific linkers and the like. For example, two overhung cohesive ends of the fragment O are A, and the length is 1nt.

Said fragment O of the invention can be made using any of the three methods as follows:
The first method comprises the steps as follows:
   1) The exogenous fragment is inserted into the poly clone sites of a gene cloning vector or gene expression vector to obtain a recombinant vector M; the nucleotide sequence of said exogenous fragment contains three recognition sequences a, b and c of the restriction endonuclease, said sequences a and c can produce unself-linked cohesive ends after the restriction endonuclease enzyme cleavage; the linking order of said three recognition sequence is a-b-c;
   2) Said target double-strand DNA fragment is inserted into said b site of said recombinant vector M to get a recombinant vector N;
   3) The recombinant vector N is enzymatically cleaved by the restrictive endonuclease recognizing said sequence a and c to get said fragment O.

Wherein, said gene cloning vector or gene expression vector may choice various vectors, such as pUC18, pUC19, pBlueScript, pQE, pGEX and pET and the like.

The second method comprises the steps as follows:
1) The exogenous fragment is inserted into the multiple cloning site in the gene cloning vector or gene expression vector to obtain a recombinant vector M; the nucleotide sequence of said exogenous fragment contains two recognition sequences a and c of the restriction endonuclease, said sequences a and c produce the unself-linked cohesive ends after the restriction endonuclease enzyme cleavage; there is a fragment b between said a and c, said fragment b contains two restriction endonuclease recognition sequences b-1 and b-2, two free basic bases of two overhung ends produced after the restriction endonuclease enzyme cleavage said sequences b-1 and b-2 are T;
2) The recombinant vector M is enzymatically cleaved by the restriction endonuclease recognizing said b-1 and b-2 to obtain a recombinant vector T;
3) The target double-strand DNA fragment is prepared, and two ends of the double-strand DNA fragment are overhung ends with free basic bases A;
4) The recombinant vector T of step 2) and the double-strand DNA fragment of step 3) are linked to obtain a recombinant vector N;
5) The recombinant vector N is enzymatically cleaved by the restriction endonuclease recognizing said sequences a and c to obtain said fragment O.

Wherein, said gene cloning vector or gene expression vector may be selected from various vectors, such as pUC 18, pUC 19, pBlueScript, pQE, pGEX and pET and the like said recognition sequences b-1 and b-1 may be same or different. The restriction endonucleases recognizing said b-1 and b-2 are some restriction endonucleases which can be directly obtained and have cohesive ends "T", such as Ahd I, BciV I, Bmr I, Xcm I and the like.

The third method comprises the steps as follows:
1) The exogenous fragment is inserted into the multiple cloning site of a gene cloning vector or gene expression vector to obtain a recombinant vector M; the nucleotide sequence of said exogenous fragment contains three recognition sequences a, b and c of the restriction endonuclease, said sequences a and c produce the unself-linked cohesive ends after the restriction endonuclease enzyme cleavage; the order of linking said three recognition sequences is a-b-c;
2) The recombinant vector M is enzymatically cleaved by the restriction endonuclease recognizing said sequence b to prepare vector T;
3) The target double-strand DNA fragment is prepared, and two ends of the double-strand DNA fragment are overhung ends with free basic bases A;
4) The vector T of step 2) and the double-strand DNA fragment of step 3) are linked to obtain a recombinant vector N;
5) The recombinant vector N is enzymatically cleaved by the restriction endonucleases recognizing said sequences a and c to obtain said fragment O.

Wherein, said gene cloning vector or gene expression vector may be selected from various vectors, such as pUC 18, pUC 19, pBlueScript, pQE, pGEX and pET and the like. To avoid the vector to self link and to facilitate the linkage of PCT products, the sequence produces planar ends after enzymatically cleaved by the restriction endonuclease recognizing said b, then adding one "T" at 3' end by Taq enzyme to form T vector; alternatively, the sequence produces cohesive ends after enzymatically cleaved by the restriction endonuclease recognizing said b, the ends are made up to planar ends, then adding one "T" at 3' end by Taq enzyme to form T vector.

In these three methods above, the cohesive ends of said sequences a and c produced through restriction endonuclease enzyme cleavage are unself-linked cohesive ends, the restriction endonuclease may be selected from various commercial endonucleases, such as, Afl III, Ahd I, Alw I, AlwN, Ava I, Ban I, Ban II, Bbs I, Bbv I, BciV I, Bgl I, Blp I, Bmr I, Bsa I, BseR I, Bsm I, BsmA I, BsmB I, BsoB I, BspM I, Bsr I, BsrD I, BstAP I, BstE II, BstF5 I, BstX I, Bsu36 I, Btg I, Bts I, Dra III, Drd I, Ear I, Eci I, EcoN I, EcoO109 I, Hga I, Hph I, Mbo II, PflF I, PflM I, Ple I, Sap I, SfaN I, Sml I, Sfi I, TspR I, Tth111 I or Xcm I and the like sold by NEB company, or some special restriction endonucleases (nick enzyme), such as Nb.Bsm I, Nb.BsrD I, Nb.bts I, Nt.Alw I and Nt.BstNB I and the like, they also can enzymatically cleave to produce unself-linked cohesive ends. In view of comprehensive consideration about multiple factors, the restriction endonuclease which can recognize 6 basic bases and enzymatically cleave to produce unself-linked cohesive ends with more than 1 basic base is preferred, such as AlwN, Bbs I, Bgl I, Blp I, Bsa I, BsmB I, BspM I, BsrD I, BstAP I, BstE II, BstX I, Bsu36 I, Bts I, Dra III, Drd I, Ear I, Eci I, PflM I, Sap I or Sfi I and the like. Among these enzymes, the restriction endonuclease which can selectively recognize 6 basic bases and enzymatically cleave to produce cohesive ends with more than 3 basic bases is more preferred, such as Bbs I, Bsa I, BsmB I, BspM I, BstE II or BstX I and the like.

Further described is another DNA molecule for expressing hairpin RNA (inverted repeat structure double-strand DNA molecule). One strand of the DNA molecule is said DNA molecule for expressing hairpin RNA, another strand is complementary to the DNA molecule for expressing hairpin RNA.

Said DNA molecule for expressing hairpin RNA can be made with the method as follows:
Said DNA molecule for expressing hairpin RNA (closed single-strand cyclic molecule) is used as a template to synthesize the DNA molecule for expressing hairpin RNA (inverted repeat structure double-strand DNA molecule) under the action of DNA polymerase with the strand displacement ability.

Wherein, there are various DNA polymerases, such as Bst DNA polymerase, Vent DNA polymerase and Klenow DNA polymerase and the like, and more specifically, Phi29 DNA polymerase.

The closed single-strand cyclic DNA molecule is amplified by a rolling circle replication mode under the action of Phi29 DNA polymerase to synthesize a great amount of double-strand DNA molecules with inverted repeat structure, and this method using the rolling circle replication mode to obtain DNA with inverted repeat structure is known as RMHR system, i.e. rolling-cycle amplification (RCA)-mediated hairpin RNA system (RMHR).

A further object of the invention is to provide a constructing method for a gene silencing vector.

The constructing method for the gene silencing vector of the invention comprises the steps as follows:
1) Said DNA molecule for expressing hairpin RNA (closed single-strand cyclic DNA) is constructed with said constructing method for the DNA molecule for expressing hairpin RNA (closed single-strand cyclic DNA);
2) Said DNA molecule for expressing hairpin RNA (inverted repeat structure double-strand DNA molecule) is constructed with said constructing method for the DNA molecule for expressing hairpin RNA (inverted repeat structure double-strand DNA molecule);
3) Said DNA molecule for expressing hairpin RNA (inverted repeat structure double-strand DNA molecule) is enzymatically cleaved by the restriction endonucleases to obtain a DNA fragment encoding the hairpin RNA, and said restriction endonucleases meet the requirement of ① or ② as follows:
   ① recognizing said recognition sequence R;
   ② recognizing said recognition sequence S;
4) The DNA fragment encoding the hairpin RNA obtained in step 3) is inserted into the multiple cloning site of the gene expression vector to obtain a gene silencing vector.

The recombinant bacteria or transgenic cell lines containing said DNA molecule for expressing hairpin RNA (inverted repeat structure double-strand DNA molecule) are also described.

### BRIEF DESCRIPTION OF DRAWINGS

Figure 1 is a schematic view for constructing the hpRNA silencing vector.
Figure 2 is the enzyme cleavage identification result of GUS gene linking to pBsa vector.
Figure 3 is the secondary structure map of the DNA fragment with the stem-loop structure: miniHP1 and miniHP2.
Figure 4 is the identification result of the inverted repeat sequence of GUS gene (hpGUS).
Figure 5 depicts the hpGUS silencing to the GUS gene.
Figure 6 is the secondary structure map of the DNA fragment with the stem-loop structure: intronHP.
Figure 7 is the enzyme cleavage identification of the gene silencing library.
Figure 8 is the secondary structure map of DNA fragment with the stem-loop structure: T hairpin.

### BEST MODES FOR CARRYNG OUT THE INVENTION

The method of constructing the DNA molecule for expressing hairpin RNA (inverted repeat structure double-strand DNA molecule) of the invention comprises the steps as follows:
(1) The hairpin DNA with asymmetric cohesive ends is designed and synthesized, the cohesive ends have to avoid the hairpin DNA to self link or avoid two different hairpin DNA to link, and the enzyme cleavage sites of restriction endonuclease are introduced into the stem or loop of the hairpin DNA in order to identify and clone the final target DNA fragment;
(2) Two ends of the target DNA fragment to be silenced are treated to be the unself-linked asymmetric cohesive ends (such as using the specific restriction endonuclease or Taq enzyme); the cohesive ends can be linked to the cohesive ends of the hairpin DNA in (1);
(3) The DNA fragments in (1) and (2) are linked to be a closed single-strand cyclic DNA molecule;
(4) The closed single-strand cyclic DNA molecule in step (3) is synthesized to the double-strand DNA molecule under the action of the DNA polymerase to form the double-strand DNA molecule with inverted repeat structure, wherein the DNA polymerase is preferentially selected from the DNA polymerase with the rolling circle replication function (such as DNA polymerase Phi29), which not only can be synthesized to the double-strand DNA, also can make the closed single-strand cyclic DNA molecule to significantly amplify.

The DNA molecule for expressing hairpin RNA (inverted repeat structure double-strand DNA molecule) can be used to construct the gene silencing vector, the double-strand DNA molecule with inverted repeat structure in step (4) above is enzymatically cleaved by enzyme and links to the expression vector to construct the gene silencing vector on the basis of obtaining the DNA molecule for expressing hairpin RNA (inverted repeat structure double-strand DNA molecule).

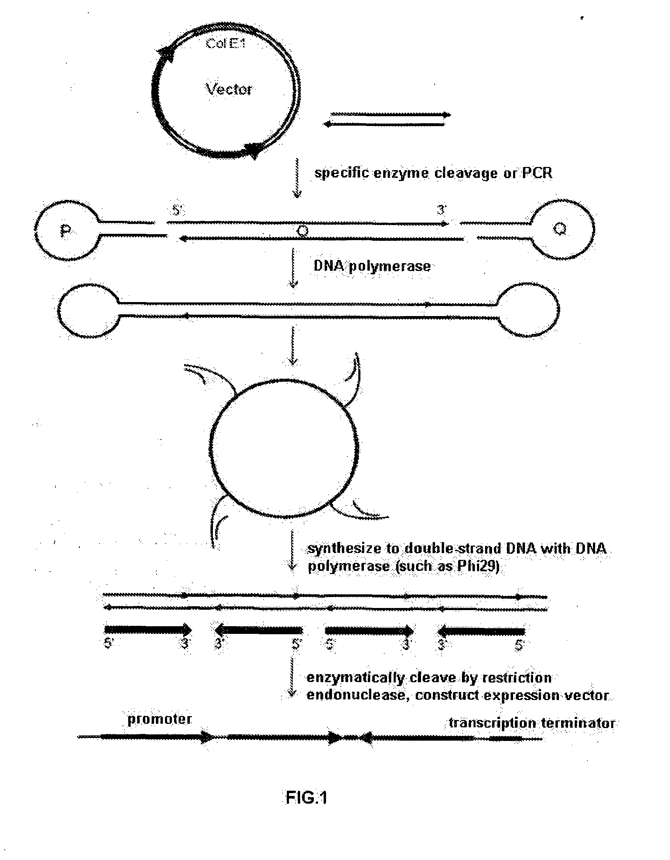

The following examples are provided to understand the invention better, but not to limit the invention. Figure 1 is a schematic view for constructing the hpRNA silencing vector.

### Example 1. Construction of the pBI121HPGUS vector for silencing GUS gene

### 1. Construction of the cloning vectors containing three restriction endonuclease recognition sequences a, b and c

Two DNA chains Bsa(+) and Bsa(-) are synthesized, the sequences of Bsa(+) and Bsa(-) are as follows:
Bsa(+): 5' GGTCTCTGGGATATCGGTCTCTGAAG 3'; (SEQ ID NO:5 in the sequence list)
Bsa(-): 5' CTTCAGAGACCGATATCCCAGAGACC 3'; (SEQ ID NO:5 in the sequence list)

Two chains Bsa(+) and Bsa(-) are annealed to form double-strand Bsa fragment. The annealing system is: Bsa(+) 50µM 1µL, Bsa(-) 50µM 1µL, 10×PCR buffer 5µL, MgCl₂ (2.5mM) 3µL, made up to the final volume of 50µL with water. The annealing conditions are: 88°C, 2 min; 65°C, 10 min; 37°C, 10 min; 25°C, 5 min.

Two DNA chains BsaT(+) and BsaT(-) are synthesized, the sequences of BsaT(+) and BsaT(-) are as follows:
BsaT(+):5'GGTCTCTGGGATATCCAAGCTTTAAATGGTTATAGCCATGGAA CTAGTGGATCCCGGGTCTCTGAAG 3'; (SEQ ID NO:7 in the sequence list)
BsaT(-):5'CTTCAGAGACCCGGGATCCACTAGTTCCATGGCTATAACCATT TAAAGCTTGGATATCCCAGAGACC 3'; (SEQ ID NO:8 in the sequence list)

Two chains BsaT(+) and BsaT(-) are annealed to form double-strand BsaT fragment, the annealing method is the same as above.

The fragments Bsa and BsaT are inserted into the vector pUC18 at SmaI site to obtain the vectors pBsa and pBsa-T respectively. The vector pBsa-T contains Bsa I and Xcm I sites, and the enzyme cleavage of Xcm I can produce a DNA vector with 3' cohesive ends being "T", which is convenience for inserting the PCR products.

### 2. The double-strand DNA fragment of GUS gene is cloned into the pBsa vector.

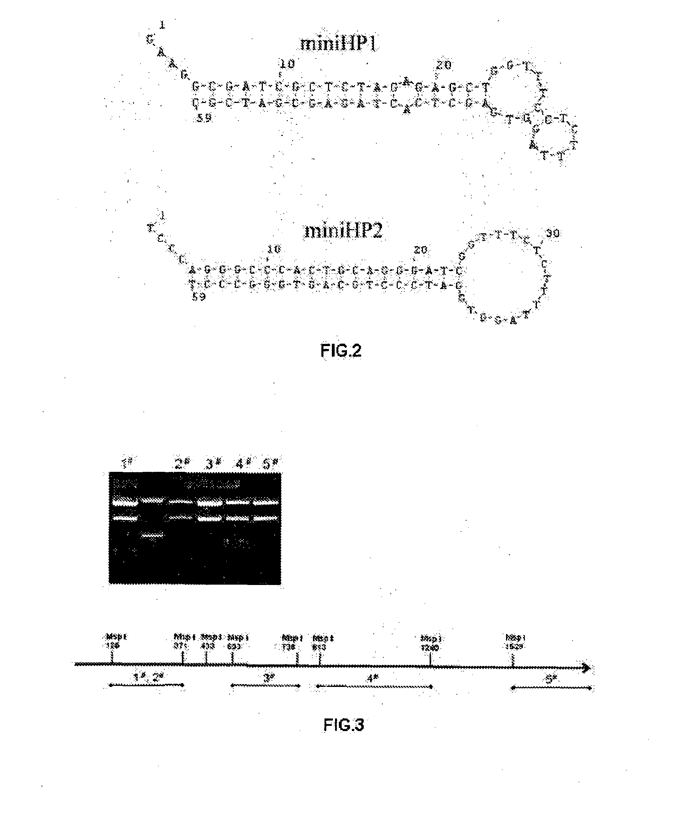

The vector pBI121 is enzymatically cleaved by enzymes BamH I and Sac I, 0.5µg 1.87kb GUS gene fragment is isolated and purified, then is enzymatically cleaved by enzyme Msp I, 0.1µg 200-600bp fragment is recovered via agarose gel electrophoresis and end filling is carried out with T4 DNA polymerase according to the instruction, then it is inserted into the vector pBsa at EcoR V site, E. coli is transformed to obtain more than 4000 clones, 10 clones is selected randomly, identified with enzyme cleavage, among others 5 clones contained the inserted target fragment, the size is 200-400bp, and the result of sequencing shows that they are evenly distributed in GUS gene from 5' to 3' end, as shown in figure 2. Wherein, the GUS gene fragment contained in pBsa-GUSF1# is 245bp and the nucleotide sequence thereof is 126-371bp region of GUS gene.

### 3. The double-strand DNA molecule with inverted repeat structure

### a) Construction of the closed single-strand cyclic DNA molecule

The DNA fragments with stem-loop structure - miniHP1 and miniHP2 are artificially synthesized and the nucleotide sequences of miniHP 1 and miniHP2 are as follows:
miniHP1 :5'-GAAGGCGATCGCTCTAGAGAGCTGGTTTCCTCTTTAGGTGA GCTCACTAGAGCGATCGC-3' (SEQ ID NO:1 in the sequence list);
miniHP2:5'-TCCCAGGGCCCACTGCAGGGATCGGTTTCTCTTTTAGGTGG ATCCATGCAGTGGGCCCT-3' (SEQ ID NO:2 in the sequence list).
miniHP1 and miniHP2 each 1 µg are annealed respectively to form the hairpin DNA, the annealing method is the same as 1. The phosphorylation is carried out according to the instruction of T4 polynucleotide kinase, 1/10 volume of 3M NaOAc and 2.5 times volume of ethanol are added to the product of phosphorylation to precipitate, the precipitate is dried and then dissolve in the sterile water to form the DNA fragment with stem-loop structure, the DNA fragment contains the cohesive ends preventing self-linkage (Figure 3).

The pBsa-GUSF1# is enzymatically cleaved by Bsa I and electrophoresed, and about 300bp GUS gene fragment is recovered for the linking reaction. The linking reaction system is: about 10ng 300bp GUS gene fragment, 10ng miniHP1, 10ng miniHP2, 2µL 10×T4 DNA ligase buffer and 0.5µL T4 DNA ligase are added into 20µl linking system. The linking reaction is carried out overnight at 16°C, then 1/10 volume of 3M NaOAc and 2.5 times volume of ethanol are added in to precipitate. The precipitate is dried and then dissolved in 20µL sterile water to form the closed single-strand cyclic DNA solution.

### b) Construction of the double-strand DNA molecule with inverted repeat structure

The closed single-strand cyclic DNA in a) is converted into the double-strand DNA molecule using DNA polymerase Phi29 with the ability of strand displacement to form the double-strand DNA molecule with inverted repeat structure.

The reaction system is as follows: 2µL 10×phi29 DNA polymerase buffer (New England Biolabs) and 1µL 100µM 6nt random primer are added into 2µL the closed single-strand cyclic DNA solution above; the mixture above is heated at 95°C for 3min and placed onto the ice for 10-15min, then 0.5µL 10mM dNTP, 0.2µL 10mg/mL BSA and 0.5µL phi29 DNA polymerase (New England Biolabs) are added in, and water is added to the final volume of 20µL.The reaction is carried out at 30°C for over 4hrs, then 1/10 volume of 3M NaOAc and 2.5 times volume of ethanol are added in to precipitate. The precipitate is dried and then dissolved in 20µL sterile water to form the solution containing the double-strand cyclic DNA with inverted repeat structure.

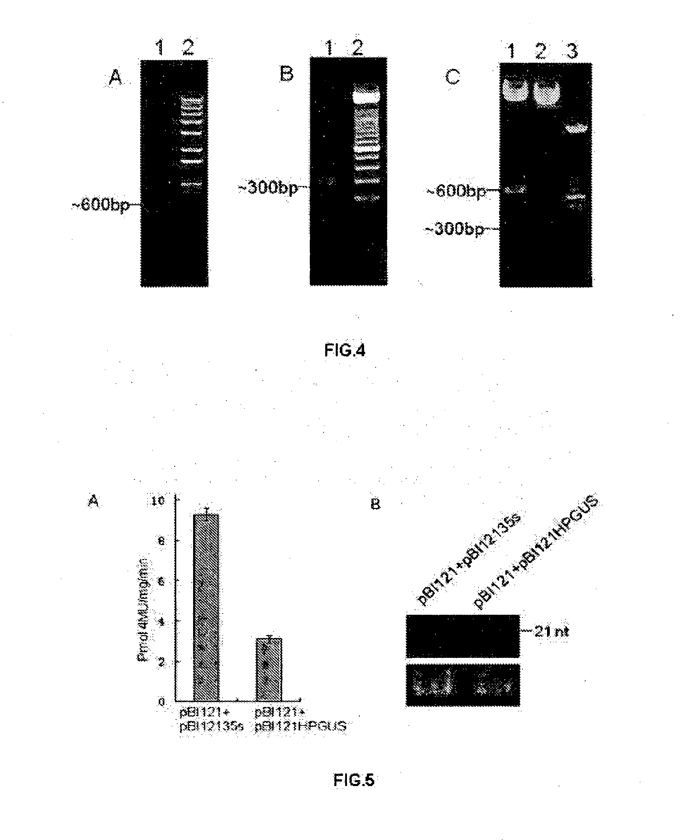

2µL double-strand DNA molecule solution above is enzymatically cleaved by Xba I, electrophoresis is carried out after 1hr, about 600bp DNA fragment is observed, the product of enzyme cleavage is further enzymatically cleaved by BamH I and electrophoresis is carried out to reveal the DNA fragment of about 300bp, whilst the band of 600bp disappears, as shown in Figure 4A and 4B. In Figure 4A, "1" represents the double-strand DNA after Xba I enzyme cleavage and amplification and "2" represents Marker; in Figure 4B, "1" represents 600bp DNA fragment of BamH I enzyme cleavage and "2" represents Marker. Because Xba I is located in miniHP1 and BamH I is located in miniHP2, the result above shows that the single-strand cyclic DNA is effectively amplified, the DNA fragment with inverted repeat structure is produced and the DNA can use to express hairpin RNA. The system based on Phi29 DNA polymerase rolling-cycle amplification method to amplify the single-strand cyclic DNA and obtain the DNA with inverted repeat structure is termed rolling-cycle mediated hairpin RNA system (RMHR system, rolling-cycle amplification (RCA)-mediated hairpin RNA system).

### 4. The construction of pBI121HPGUS vector

The product of amplification above is enzymatically cleaved by Xba I and Sac I, about 600bp DNA fragment is isolated and purified through the agarose electrophoresis, pBI121 vector is also enzymatically cleaved by Xba I and Sac I and about 11kb fragment of the vector is recovered. The enzyme-link system is as follows: 20ng 600bp DNA, 10ng pBI121 vector, 1µL 10× T4 polymerase buffer, 0.5µL T4 DNA ligase, and water is added to 10µL, the link reaction is carried out overnight at 16°C, *E.coli* is transformed, the correct clone is identified with enzyme cleavage, and GUS gene silencing vector pBI121 HPGUS is obtained; the result of enzyme cleavage identification of pBI121HPGUS is shown in Figure 4C. In figure 4C, "1" represents pBI121HPGUS vector with Xba I and Sac I enzyme cleavage, and about 600bp fragment is enzymatically cleaved from pBI121HPGUS vector; "2" represents pBI121 HPGUS vector with three enzymes Sac I, BamH I and Xba I enzyme cleavage, and about 300bp fragment is enzymatically cleaved from pBI121HPGUS, which indicate that the sequence inserted has inverted repeat structure, and the result is further confirmed through DNA sequencing.

### 5. The effect of pBI121HPGUS silencing vector on the expression of GUS gene

pBI121HPGUS and pBI121 are transferred into *Agrobacterium* GV310 respectively, the pBI121 vector is self linked after enzymatically cleaved by Sma I and Ec1136II to obtain the vector pBI121-35S, and the vector pBI121-35S is also transferred into GV310 as control.

*Nicotiana benthamiana* grows at about 24°C and 16h light/8h dark, and uses to perform Agrobacterium injection experiment when it grows to 8-10 leaves. The method of *Agrobacterium* injection experiment is as follows: the single *Agrobacterium* clones containing pBI121HPGUS, pBI121 and pBI121-35S are picked, inoculated into 3mL YEB medium (with 50mg/L rifamycin and 50mg/L kanamycin), incubated overnight at 28°C and then transferred into 15ml YEB medium (with 50mg/L rifamycin, 50mg/L kanamycin, 10mM MES and 20mM acetosyringone), and incubated at 28°C for 8 hrs, then the thallus is collected and resuspended in the injection buffer (10mM MgCl₂, 10mM MES and 200mM acetosyringone), OD₆₀₀ is adjusted to 2.0, it is settled at the room temperature for 2∼3hrs. 4mL pBI121HPGUS *Agrobacterium* and 1mL pBI121 *Agrobacterium* are mixed uniformly, and injected into the leaves of *Nicotiana benthamiana* with 1mL disposable injector. pBI121-35S and pBI121 *Agrobacterium* are mixed uniformly and injected into *Nicotiana benthamiana* as control by the same method as above. The injected plants grow under the same culture condition for other 48hrs, and the GUS activity quantitative analysis and Northern blot hybridization are performed.

The method of the GUS activity quantitative analysis is as follows:
50mg tobacco's leaves injected *Agrobacterium* are milled to powder with liquid nitrogen, 0.5mL protein extracting buffer is added (50mM Na₂H₂PO₄, 10 mM EDTA, 0.1% Triton X-100 and 1.0g L⁻¹ lauroyl sarcosine sodium), they are centrifuged at 10000g for 5min after mixing uniformly, the supernatant is recovered for GUS activity measurement. The measurement of the total protein concentration is referred to Bradford's method (Bradford, M.M. (1976) Anal Biochem72, 248-254). The measurement of GUS activity is performed using MUG as substrate; the specific measurement is referred to the article of Jefferson et. al. (Jefferson, R.A., EMBO J (1987) 6, 3901-3907). The apparatus for measurement is Hoefer DyNA Quant 200 from Amersham Biosciences Company, and the operational procedure is performed according to the operating instruction provided.

The result of GUS activity quantitative analysis is shown in 5A, indicating that the GUS activity in the leaves injected pBI121HPGUS and pBI121 is significantly lower than the GUS activity in the leaves injected pBI121-35S and pBI121.

The method of Northern blot hybridization is as follows: 100mg tobacco's leaves injected *Agrobacterium* is milled to powder with liquid nitrogen, 1 mL Trizol (Invitrogen) is added, the total RNA is exacted and the operational procedure is performed according to the instruction. The isolation, electrophoresis and transferring to a membrane of the total RNA are carried out according to Wang's article (Wang, L., FEBS Lett (2007) 581, 3848-3856). pBsa-GUSF1# plasmid is enzymatically cleaved by Bsa I and about 300bp GUS DNA fragment is recovered as probe, the labeling kit is Prime-a-Gene Labeling System (Promega), ³²P-dCTP label. The method of hybridization is performed according to the article of Wang L. et. al. (Wang, L., FEBS Lett (2007) 581, 3848-3856). The sign of hybridization is detected using the autoradiography.

The result of Northern blot hybridization is shown in Figure 5B, there is about 22nt siRNA band in the RNA of the leaves injected pBI121HPGUS and pBI121, whereas there is no siRNA sign in the RNA of the leafves injected pBI121-35S and pBI121.

The results of the experiments above indicate that the silencing vector pBI121HPGUS constructed can produce siRNA silencing GUS gene, which silence the GUS gene expressed by pBI121 vector.

### Example 2. The construction of Arabidopsis hairpin RNA silencing library 1. The construction of Arabidopsis cDNA library based on pBsa-T vector

The *Arabidopsis* cDNA library is purchased from ABRC (Arabidopsis Biological Resource Center , Stock Number CD4-7) and the amplification of the library and the extracting method of the plasmid are referred to the instruction provided by ABRC.

10µg library's cDNA is extracted, the cDNA fragment is enzymatically cleaved from the vector by Not I and Sal I, about 1µg 400-2000bp DNA fragment is recovered, then the DNA fragment recovered is enzymatically cleaved by Alu I, the product of enzyme cleavage is precipitated with 1/10 volume of 3M NaOAC and 2.5 times volume of ethanol, the precipitate is dried and dissolved in 20µl sterile water; 3µl 10×PCR buffer, 0.5µl 10mM dATP and 1U Taq are added, and water is added to 30µL, they react at 72°C for 30min, "A" are added at the 3' end of the product of enzyme cleavage. About 100ng 100-1000bp Arabidopsis cDNA fragment is recovered through the agarose electrophoresis.

pBsa-T vector is enzymatically cleaved by Xcm I and the pBsa-T vector obtained is used to perform the linking reaction, the system of linking reaction is as follows: 20ng *Arabidopsis* cDNA fragment recovered, 10ng pBsa-T vector, 2µL 10×T4 DNA ligase, 0.5µL ligase, and water is added to 20µL. The linking reaction is carried out at 16°C overnight. The product of linkage is used to transform *E.coli* to obtain *Arabidopsis* cDNA library pBsa-Atlib containing 10⁶ clones. The library obtained is amplified, and the method of amplification is performed according to the instruction of SuperScriptTM Plasmid System for cDNA Synthesis and Plasmid Cloning Kit from GibcoBRL Company. The plasmid DNA is exacted from the library amplified, 3µg DNA is taken and enzymatically cleaved by Bsa I, and about 100ng 100-1000bp DNA fragment is recovered through the agarose electrophoresis.

### 2. Obtaining the closed single-strand cyclic Arabidopsis cDNA

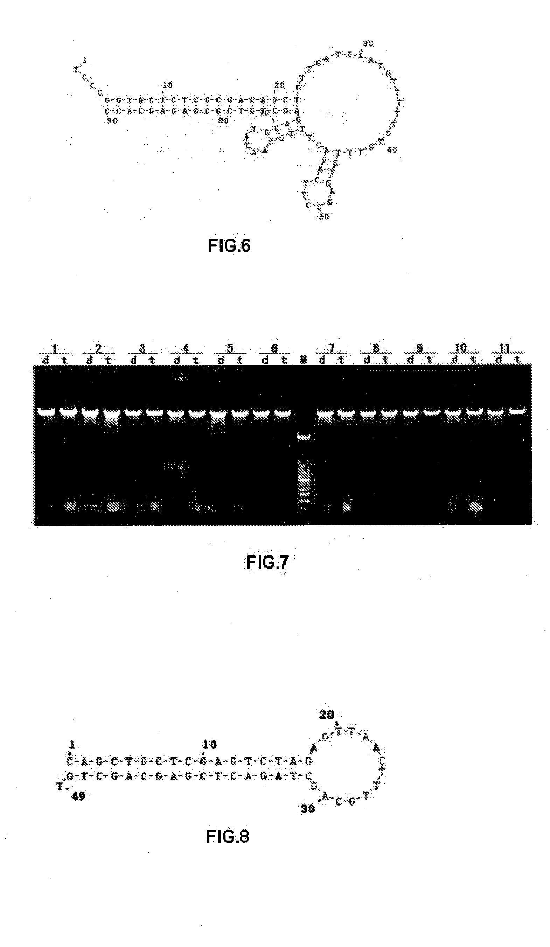

The intronHP DNA sequence with the intron is designed and synthesized in order to improve the silencing efficiency of gene and enhance the stability of the inverted repeat sequence in bacteria, and the sequence of intronHP is as follows: 5'TCCCGGTGCTCTCGCGACAGCTGTTGATCAATGTTTTTGTGTTTCTGAGTC TTCAGACTTTGCAATATGCAGAGCTGTCGCGAGAGCACC3'(SEQ ID NO:3 in the sequence list), the DNA strand is self-annealed to form the hairpin DNA structure with the intron (Figure 6), and the method is the same as the step 3 of Example 1.

10ng 100-1000bp DNA fragment recovered is taken and added with 10ng miniHP1 or 10ng intronHP and 2µl 10×T4 ligase reaction buffer and 0.5µl T4 DNA ligase , then water is added to 20µL, the linking reaction is carried out at 16°C overnight, then 1/10 volume of 3M NaOAC and 2.5 times volume of ethanol are added to precipitate, the precipitate is dried and dissolved in 20µl sterile water to obtain the closed single-strand cyclic DNA.

### 3. Construction of the double-strand DNA molecule with inverted repeat structure

The single-strand cyclic DNA is amplified with Phi29 DNA polymerase and the reaction system is as follows: 2µL single-strand cyclic DNA solution above is taken, 2µL 10×Phi29 DNA polymerase buffer and 1µL 100µM 6nt random primer are added in; and the mixture as above is heated at 95°C for 3mins, and then placed on the ice for 10-15mins, 0.5µL 10mM dNTP, 0.2µL 10mg/mL BSA and 0.5µL phi29 DNA polymerase are added in, and water is added to the final volume of 20µL.

The reaction system above is reacted at 30°C for more than 4hrs, then added 1/10 volume of 3M NaOAC and 2.5 times volume of ethanol to precipitate, the precipitate is dried and dissolved in 30µl sterile water to obtain *Arabidopsis's* double-strand DNA with inverted repeat structure.

### 4. Obtaining Arabidopsis hairpin RNA silencing library pBI121-hpRNA

4µL 10×enzyme cleavage buffer, 1µL Xba I and 1µL Sac I are added into the double-strand DNA solution above, water is added to 40µL, the enzyme cleavage reaction is performed at 37°C for 2hrs, 200-2000bp DNA fragment is recovered though electrophoresis and links with pBI121 vector which is enzymatically cleaved by Xba I and Sac I and recovered to obtain the recombinant plasmid pBI121-hpRNA, pBI121-hpRNA is used to transform *E. coli* to get 10⁵ clones, 12 clones are randomly selected and identified with Xba I and Sac I enzyme cleavage, wherein 11 clones are the recombinant plasmid pBI121-hpRNA, indicating that above 90% clones of the library constructed have inserted the target DNA fragment, pBI121-cDNA is enzymatically cleaved by Xba I, Sac I and Pvu II and the inserted fragments are reduced half, indicating that the inserted fragments are the inverted repeat structure, as shown in Figure 7. In Figure 7, "d" represents Xba I and Sac I enzyme cleavage; "t" represents Xba I, Sac I and PvuII enzyme cleavage, "1-11" represents 11 clones containing pBI121-hpDNA and "M" represents Marker. 20 single clones are randomly picked and experienced DNA sequencing, the result of sequencing is shown that above 95% sequences of the hairpin library constructed come from *Arabidopsis*'s cDNA and have the inverted repeat structure, the product expressed can form the haipin RNA. The results above indicate that the hairpin RNA silencing/RNAi library of *Arabidopsis* is successfully constructed using the RMHR method above (the step 3 of Example 1).

### Example 3. Constructing hairpin RNA vector with PCR product

### 1. Obtaining short hairpin DNA

The single-strand DNA-T hairpin is artificially synthesized and the nucleotide sequence thereof is as follows:
T hairpin: 5'cagctgctcgagtctagagttaaCtttgcagCTAGACTCGAGCAGCTGt 3' (SEQ ID NO:4 in the sequence list).

The sequences are annealed to form the hairpin DNA with stem-loop structure, the method is the same as the step 3 of Example 1, and 3' end of stem of the hairpin DNA is cohesive end with "T" (Figure 8).

### 2. Obtaining the PCR product

The primers GUSfw and GUSrv are synthesized and their sequences are as follows:
GUSfw : 5' AAGCTTCGGGCAATTGCTGTGCCAG3'(SEQ ID NO:9 in the sequence list);
GUSrv : 5' CGGCAATAACATACGGCGTG3'(SEQ ID NO:10 in the sequence list).

The GUS gene is amplified using GUSfw and GUSrvₒ

PCR system: 1ng pBI121, 5µL 10×buffer, 10µM GUSfw and GUSrv each 2µL, 1µL 10mM dNTP, 1U Pfu, and water is added to 50µL.

PCR condition: 94°C 3min, 94°C 30sec, 55°C 30sec, 72°C 30sec, 30 cycles, to obtain 246 bp DNA fragment of 127-373bp of GUS gene.

1/10 volume of 3M NaOAC and 2.5 times volume of ethanol are added in the PCR product to precipitate, the precipitate is dried and dissolved in 20µL sterile water; 3µL 10×buffer and 0.5µL 10mM dATP, 1U Taq are added in, then water is added to 30µL, reaction is carried out at 72°C for 30min to add "A" at 3' end of the PCR product. The product is experienced the agarose electrophoresis to recover about 250bp DNA fragment.

### 3. Obtaining the closed single-strand cyclic DNA

About 20ng 250bp GUS gene fragment, 10ng T hairpin, 2µL 10×T4 ligase reaction buffer, 0.5µL T4 ligase are added into 20µL linking system. The linking reaction is carried out at 16°C overnight, 1/10 volume of 3M NaOAC and 2.5 times volume of ethanol are added in the product to precipitate, the precipitate is dried and dissolved in 20µL sterile water to obtain the closed single-strand cyclic DNA solution.

### 4. Construction of the double-strand DNA molecule with inverted repeat structure

The single-strand cyclic DNA obtained above is amplified with Phi29 DNA ligase, and the amplification method and the reaction system are the same as Example 1. The product amplified is experienced the agarose electrophoresis, and the large fragment of DNA product with inverted repeat structure is observed, the product is enzymatically cleaved by HindIII to produce about 550bp DNA fragment, the fragment may be further enzymatically cleaved by Xba I to produce about 250bp DNA fragment, and the results above indicate that the double-strand DNA molecule with inverted repeat structure is obtained using the RMHR method as above (the step 3 in Example 1)

### Industry application

Described is a DNA molecule for expressing hairpin RNA, and the invention provides a constructing method and application thereof. The invention firstly constructs a closed single-strand cyclic DNA molecule, the DNA molecule is used as the template, the closed single-strand cyclic DNA molecule is amplified using DNA polymerase such as Phi29 to form the double-strand DNA molecule with inverted repeat structure, the double-strand DNA molecule can produce DNA with inverted repeat structure only with suitable enzyme cleavage, the DNA can produce RNA with hairpin structure after transcription, thus the method has the characteristics of rapidness, high-throughput and low cost, and can be used to construct the gene silencing vector with hairpin structure with various length to silence one and more target genes. The long hairpin RNA (lhRNA) library has the application potential in exploiting and identifying gene function and the like areas, there is still no report about the construction method for long hairpin RNA (lhRNA) whose length of stem is above 40bp, the construction method for the DNA molecule for expressing hairpin RNA provided by the invention has the function which can not be replaced in constructing the long hairpin RNA (lhRNA) library, the DNA from various biologic sources such as animals, plants and microorganisms can construct the gene silencing/RNAi vector or library.

## Claims

1. A method for constructing a DNA molecule, wherein three DNA fragments O, P and Q are linked to a closed single-strand cyclic molecule;
Said fragment O is a target double-strand DNA fragment with two cohesive ends which can not be self linked,
Said fragments P and Q are DNA fragments with stem-loop structure having cohesive ends respectively, the cohesive ends of said fragments P and Q are matched with the two cohesive ends of said fragment O, and said fragments P and Q meet the requirement of i) or ii) as follows:
i) the nucleotide sequence of fragment P or Q comprises at least one sequence e, said sequence e forms a recognition sequence R of restriction endonuclease with the complemented sequence thereof;
ii) the nucleotide sequence of said fragment P comprises at least one sequence e, said sequence e forms a recognition sequence R of restriction endonuclease with the complemented sequence thereof; the nucleotide sequence of said fragment Q comprises at least one sequence f, said sequence f forms a recognition sequence S of restriction endonuclease with the complemented sequence thereof; said recognition sequence R and S are recognized by the different restriction endonucleases.

2. The method for constructing the DNA molecule according to claim 1, wherein said fragment O is obtained according to the method as follows:
1) The exogenous fragment is inserted into the poly clone sites of a gene cloning vector or gene expression vector to obtain a recombinant vector M; the nucleotide sequence of said exogenous fragment contains three recognition sequences a, b and c of the restriction endonuclease, said sequences a and c can produce unself-linked cohesive ends after the restriction endonuclease enzyme cleavage; the linking order of said three recognition sequence is a-b-c;
2) Said target double-strand DNA fragment is inserted into said b site of said recombinant vector M to get a recombinant vector N;
3) The recombinant vector N is enzymatically cleaved by the restrictive endonuclease recognizing said sequence a and c to get said fragment O.

3. The method for constructing the DNA molecule according to claim 1, wherein said fragment O is obtained according to the method as follows:
1) The exogenous fragment is inserted into the multiple cloning site in the gene cloning vector or gene expression vector to obtain a recombinant vector M; the nucleotide sequence of said exogenous fragment contains two recognition sequences a and c of the restriction endonuclease, said sequences a and c produce the unself-linked cohesive ends after the restriction endonuclease enzyme cleavage; there is a fragment b between said a and c, said fragment b contains two restriction endonuclease recognition sequences b-1 and b-2, two free basic bases of two overhung ends produced after the restriction endonuclease enzyme cleavage said sequences b-1 and b-2 are T;
2) The recombinant vector M is enzymatically cleaved by the restriction endonuclease recognizing said b-1 and b-2 to obtain a recombinant vector T;
3) The target double-strand DNA fragment is prepared, and two ends of the double-strand DNA fragment are overhung ends with free basic bases A;
4) The recombinant vector T of step 2) and the double-strand DNA fragment of step 3) are linked to obtain a recombinant vector N;
5) The recombinant vector N is enzymatically cleaved by the restriction endonuclease recognizing said sequences a and c to obtain said fragment O.

4. The method for constructing the DNA molecule according to claim 1, wherein said fragment O is obtained according to the method as follows:
1) The exogenous fragment is inserted into the multiple cloning site of a gene cloning vector or gene expression vector to obtain a recombinant vector M; the nucleotide sequence of said exogenous fragment contains three recognition sequences a, b and c of the restriction endonuclease, said sequences a and c produce the unself-linked cohesive ends after the restriction endonuclease enzyme cleavage; the order of linking said three recognition sequences is a-b-c;
2) The recombinant vector M is enzymatically cleaved by the restriction endonuclease recognizing said sequence b to prepare vector T;
3) The target double-strand DNA fragment is prepared, and two ends of the double-strand DNA fragment are overhung ends with free basic bases A;
4) The vector T of step 2) and the double-strand DNA fragment of step 3) are linked to obtain a recombinant vector N;
5) The recombinant vector N is enzymatically cleaved by the restriction endonucleases recognizing said sequences a and c to obtain said fragment O.

5. The method for constructing the DNA molecule according to claim 2, 3 or 4, wherein the sequences of said a and c produce the unself-linked cohesive ends with at least one overhung basic base after restriction endonucleases enzyme cleavage.

6. The method for constructing the DNA molecule according to claim 5, wherein the sequences of said a and c produce the unself-linked cohesive ends with at least three overhung basic bases after restriction endonucleases enzyme cleavage.

7. A method for constructing a gene silencing vector, which comprises the steps as follows:
1) The DNA molecule for expressing hairpin RNA obtained by the method of any one of claims 1 to 6 is constructed with the constructing method according to any one of claims 1 to 6;
2) The DNA molecule for expressing hairpin RNA, which is a double-strand DNA, one strand is the DNA molecule obtained by the method of any one of claims 1 to 6, another strand is complementary to the DNA molecule obtained by the method of any one of claims 1 to 6, is constructed with the constructing method wherein the DNA molecule obtained by the method of any one of claims 1 to 6 is used as a template to synthesize the complementary strand thereof under the action of DNA polymerase with the strand displacement ability to form the DNA molecule for expressing hairpin RNA, which is a double-strand DNA, one strand is the DNA molecule obtained by the method of any one of claims 1 to 6, another strand is complementary to the DNA molecule obtained by the method of any one of claims 1 to 6, optionally wherein said DNA polymerase is phi29 DNA polymerase;
3) The DNA molecule for expressing hairpin RNA, which is a double-strand DNA, one strand is the DNA molecule obtained by the method of any one of claims 1 to 6, another strand is complementary to the DNA molecule obtained by the method of any one of claims 1 to 6 is enzymatically cleaved by the restriction endonucleases to obtain a DNA fragment encoding the hairpin RNA, and said restriction endonucleases meet the requirement of ① or ② as follows:
① recognizing said recognition sequence R;
② recognizing said recognition sequence S;
4) The DNA fragment encoding the hairpin RNA obtained in step 3) is inserted into the multiple cloning site of the gene expression vector to obtain a gene silencing vector.

8. The use of the constructing method according to claim 7 in the construction of hairpin RNA library.

## Patentansprüche

1. Verfahren zum Konstruieren eines DNA-Moleküls, wobei drei DNA-Fragmente 0, P und Q zu einem geschlossenen einsträngigen zyklischen Molekül verkettet werden;
das Fragment O ein doppelsträngiges Target-DNA-Fragment mit zwei kohäsiven Enden ist, die nicht mit sich selbst verkettet sein können,
die Fragmente P und Q DNA-Fragmente mit einer Haarnadelstruktur sind, die jeweils kohäsive Enden aufweisen, wobei die kohäsiven Enden der Fragmente P und Q mit den zwei kohäsiven Enden des Fragments O zusammen passen, und die Fragmente P und Q der Anforderung i) oder ii) wie folgt genügen:
i) die Nukleotidsequenz von Fragment P oder Q enthält wenigstens eine Sequenz e, wobei die Sequenz e eine Erkennungssequenz R von Restriktionsendonuklease mit der komplementierten Sequenz davon bildet;
ii) die Nukleotidsequenz des Fragments P enthält wenigstens eine Sequenz e, wobei die Sequenz e eine Erkennungssequenz R von Restriktionsendonuklease mit der komplementierten Sequenz davon bildet; die Nukleotidsequenz des Fragments Q enthält wenigstens eine Sequenz f, wobei die Sequenz f eine Erkennungssequenz S von Restriktionsendonuklease mit der komplementierten Sequenz davon bildet; wobei die Erkennungssequenzen R und S durch die unterschiedlichen Restriktionsendonukleasen erkannt werden.

2. Verfahren zum Konstruieren des DNA-Moleküls nach Anspruch 1, wobei das Fragment 0 gemäß dem folgenden Verfahren erhalten wird:
1) Das exogene Fragment wird in die Polyklonstellen eines Genklonierungsvektors oder Genexpressionsvektors eingebracht, um einen rekombinanten Vektor M zu erhalten; die Nukleotidsequenz des exogenen Fragments enthält drei Erkennungssequenzen a, b und c der Restriktionsendonuklease, wobei die Sequenzen a und c nach der Restriktionsendonukleasenenzym-Spaltung nicht mit sich selbst verkettete kohäsive Enden erzeugen können; die Verkettungsreihenfolge der drei Erkennungssequenzen ist a-b-c;
2) Das doppelsträngige Target-DNA-Fragment wird in die b-Stelle des rekombinanten Vektors M eingebracht, um einen rekombinanten Vektor N zu erhalten;
3) Der rekombinante Vektor N wird enzymatisch durch die restriktive Endonuklease gespalten, welche die Sequenz a und c erkennt, um das Fragment O zu erhalten.

3. Verfahren zum Konstruieren des DNA-Moleküls nach Anspruch 1, wobei das Fragment 0 nach dem folgenden Verfahren erhalten wird:
1) Das exogene Fragment wird in die multiple Klonierungsstelle im Genklonierungsvektor oder Genexpressionsvektor eingebracht, um einen rekombinanten Vektor M zu erhalten; die Nukleotidsequenz des exogenen Fragments enthält zwei Erkennungssequenzen a und c der Restriktionsendonuklease, wobei die Sequenzen a und c die nicht mit sich selbst verketteten kohäsiven Enden nach der Restriktionsendonukleasenenzym-Spaltung erzeugen; es gibt ein Fragment b zwischen dem a und c, wobei das Fragment b zwei Restriktionsendonukleasenerkennungssequenzen b-1 und b-2 enthält, zwei freie Grundbasen von zwei überstehenden Enden, die nach der Restriktionsendonukleasenenzym-Spaltung erzeugt wurden, wobei die Sequenzen b-1 und b-2 T sind;
2) Der rekombinante Vektor M wird enzymatisch durch die Restriktionsendonuklease gespalten, welche b-1 und b-2 erkennt, um einen rekombinanten Vektor T zu erhalten;
3) Das doppelsträngige Target-DNA-Fragment wird hergestellt, und zwei Enden des doppelsträngigen DNA-Fragments sind überstehende Enden mit freien Grundbasen A;
4) Der rekombinante Vektor T von Schritt 2) und das doppelsträngige DNA-Fragment von Schritt 3) werden verkettet, um einen rekombinanten Vektor N zu erhalten;
5) Der rekombinante Vektor N wird enzymatisch durch die Restriktionsendonuklease gespalten, welche die Sequenzen a und c erkennt, um das Fragment 0 zu erhalten.

4. Verfahren zum Konstruieren des DNA-Moleküls nach Anspruch 1, wobei das Fragment 0 gemäß dem folgenden Verfahren erhalten wird:
1) Das exogene Fragment wird in die multiple Klonierungsstelle eines Genklonierungsvektors oder Genexpressionsvektors eingebracht, um einen rekombinanten Vektor M zu erhalten; die Nukleotidsequenz des exogenen Fragments enthält drei Erkennungssequenzen a, b und c der Restriktionsendonuklease, wobei die Sequenzen a und c die nicht mit sich selbst verketteten kohäsiven Enden nach der Restriktionsendonukleasenenzym-Spaltung erzeugen; die Verkettungsreihenfolge der drei Erkennungssequenzen ist a-b-c;
2) Der rekombinante Vektor M wird enzymatisch durch die Restriktionsendonuklease gespalten, welche die Sequenz b erkennt, um den Vektor T herzustellen;
3) Das doppelsträngige Target-DNA-Fragment wird hergestellt, und zwei Enden des doppelsträngigen DNA-Fragments sind überstehende Enden mit freien Grundbasen A;
4) Der Vektor T von Schritt 2) und das doppelsträngige DNA-Fragment von Schritt 3) werden verkettet, um einen rekombinanten Vektor N zu erhalten;
5) Der rekombinante Vektor N wird enzymatisch durch die Restriktionsendonukleasen gespalten, welche die Sequenzen a und c erkennen, um das Fragment O zu erhalten.

5. Verfahren zum Konstruieren des DNA-Moleküls nach Anspruch 2, 3 oder 4, wobei die Sequenzen von a und c die nicht mit sich selbst verketteten kohäsiven Enden mit wenigstens einer überstehenden Grundbase nach der Restriktionsendonukleasenenzym-Spaltung erzeugen.

6. Verfahren zum Konstruieren des DNA-Moleküls nach Anspruch 5, wobei die Sequenzen von a und c die nicht mit sich selbst verketteten kohäsiven Enden mit wenigstens drei überstehenden Grundbasen nach der Restriktionsendonukleasenenzym-Spaltung erzeugen.

7. Verfahren zum Konstruieren eines Gen-Stilllegungsvektors, welches die folgenden Schritte umfasst:
1) Das DNA-Molekül zum Exprimieren von Haarnadel-RNA, erhalten durch das Verfahren nach einem der Ansprüche 1 bis 6, wird mit dem Konstruktionsverfahren nach einem der Ansprüche 1 bis 6 konstruiert;
2) Das DNA-Molekül zum Exprimieren von Haarnadel-RNA, die eine doppelsträngige DNA ist, wobei ein Strang das DNA-Molekül ist, das durch das Verfahren nach einem der Ansprüche 1 bis 6 erhalten wird, und ein anderer Strang komplementär zum DNA-Molekül ist, das nach dem Verfahren nach einem der Ansprüche 1 bis 6 erhalten wird, wird mit dem Verfahren zum Konstruieren konstruiert, wobei das DNA-Molekül, das durch das Verfahren nach einem der Ansprüche 1 bis 6 erhalten wird, als Template zum Synthetisieren des komplementären Stranges davon unter der Wirkung von DNA-Polymerase mit der Strangverdrängungsfähigkeit verwendet wird, um das DNA-Molekül zum Exprimieren von Haarnadel-RNA zu bilden, die eine doppelsträngige DNA ist, wobei ein Strang das DNA-Molekül ist, das durch das Verfahren nach einem der Ansprüche 1 bis 6 erhalten wird, und wobei ein anderer Strang komplementär zum DNA-Molekül ist, das durch das Verfahren nach einem der Ansprüche 1 bis 6 erhalten wird, wobei optional die DNA-Polymerase phi29-DNA-Polymerase ist;
3) Das DNA-Molekül zum Exprimieren von Haarnadel-RNA, die eine doppelsträngige DNA ist, wobei ein Strang das DNA-Molekül ist, das durch das Verfahren nach einem der Ansprüche 1 bis 6 erhalten wird, und wobei ein weiterer Strang komplementär zum DNA-Molekül ist, das durch das Verfahren nach einem der Ansprüche 1 bis 6 erhalten wird, wird enzymatisch durch die Restriktionsendonukleasen gespalten, um ein DNA-Fragment zu erhalten, das die Haarnadel-RNA kodiert, und wobei die Restriktionsendonukleasen der Anforderung ① oder ② wie folgt genügen:
① Erkennen der Erkennungssequenz R;
② Erkennen der Erkennungssequenz S;
4) Das DNA-Fragment, das die in Schritt 3) erhaltene Haarnadel-RNA kodiert, wird in die multiple Klonierungsstelle des Genexpressionsvektors eingebracht, um einen Gen-Stilllegungsvektor zu erhalten.

8. Verwenden des Konstruktionsverfahrens nach Anspruch 7 zum Konstruieren einer Haarnadel-RNA-Bibliothek.

## Revendications

1. Procédé de construction d'une molécule d'ADN, dans lequel trois fragments d'ADN O, P et Q sont liés à une molécule cyclique simple brin fermée ;
ledit fragment O étant un fragment d'ADN double brin cible ayant deux extrémités cohésives ne pouvant se lier l'une à l'autre,
lesdits fragments P et Q étant des fragments d'ADN ayant une structure tige-boucle ayant respectivement des extrémités cohésives, les extrémités cohésives desdits fragments P et Q étant appariées aux deux extrémités cohésives dudit fragment O, et lesdits fragments P et Q satisfaisant à l'exigence i) ou ii) ci-après :
i) la séquence nucléotidique du fragment P ou Q comprend au moins une séquence e, ladite séquence e formant une séquence de reconnaissance R d'une endonucléase de restriction avec sa séquence complémentaire ;
ii) la séquence nucléotidique dudit fragment P comprend au moins une séquence e, ladite séquence e formant une séquence de reconnaissance R d'une endonucléase de restriction avec sa séquence complémentaire ; la séquence nucléotidique dudit fragment Q comprend au moins une séquence f, ladite séquence f formant une séquence de reconnaissance S d'une endonucléase de restriction avec sa séquence complémentaire ; lesdites séquences de reconnaissance R et S étant reconnues par les endonucléases de restriction différentes.

2. Procédé de construction d'une molécule d'ADN selon la revendication 1, dans lequel ledit fragment O est obtenu par le procédé suivant :
1) le fragment exogène est inséré dans les sites de polyclonage d'un vecteur de clonage de gènes ou d'un vecteur d'expression de gènes pour obtenir un vecteur recombinant M ; la séquence nucléotidique dudit fragment exogène contient trois séquences de reconnaissance a, b et c de l'endonucléase de restriction, lesdites séquences a et c peuvent produire des extrémités cohésives non liées l'une à l'autre après clivage par une enzyme endonucléase de restriction ; l'ordre de liaison desdites trois séquences de reconnaissance est a-b-c ;
2) ledit fragment d'ADN double brin cible est inséré dans ledit site b dudit vecteur recombinant M pour donner un vecteur recombinant N ;
3) le vecteur recombinant N est soumis à un clivage enzymatique par l'endonucléase de restriction reconnaissant lesdites séquences a et c, pour donner ledit fragment O.

3. Procédé de construction de la molécule d'ADN selon la revendication 1, dans lequel ledit fragment O est obtenu par le procédé suivant :
1) le fragment exogène est inséré dans le site de polyclonage du vecteur de clonage de gènes ou du vecteur d'expression de gènes pour obtenir un vecteur recombinant M ; la séquence nucléotidique dudit fragment exogène contient deux séquences de reconnaissance a et c de l'endonucléase de restriction, lesdites séquences a et c produisent les extrémités cohésives non liées l'une à l'autre après clivage par l'enzyme endonucléase de restriction ; il y a un fragment b entre ledit a et ledit c, ledit fragment b contient deux séquences de reconnaissance d'endonucléases de restriction b-1 et b-2, deux bases basiques libres de deux extrémités en surplomb produites après clivage par une enzyme endonucléase de restriction desdites séquences b-1 et b-2, sont T ;
2) le vecteur recombinant M est soumis à un clivage enzymatique par l'endonucléase de restriction reconnaissant lesdits b-1 et b-2 pour obtenir un vecteur recombinant T ;
3) le fragment d'ADN double brin cible est préparé, et deux extrémités du fragment d'ADN double brin sont des extrémités en surplomb comportant des bases basiques libres A ;
4) le vecteur recombinant T de l'étape 2) et le fragment d'ADN double brin de l'étape 3) sont liés pour obtenir un vecteur recombinant N ;
5) le vecteur recombinant N est soumis à un clivage enzymatique par l'endonucléase de restriction reconnaissant lesdites séquences a et c, pour obtenir ledit fragment O.

4. Procédé de construction de la molécule d'ADN selon la revendication 1, dans lequel ledit fragment O est obtenu par le procédé suivant :
1) le fragment exogène est inséré dans le site de polyclonage d'un vecteur de clonage de gènes ou d'un vecteur d'expression de gènes pour obtenir un vecteur recombinant M ; la séquence nucléotidique dudit fragment exogène contient trois séquences de reconnaissance a, b et c de l'endonucléase de restriction, lesdites séquences a et c produisent les extrémités cohésives non liées l'une à l'autre après clivage par une enzyme endonucléase de restriction ; l'ordre de liaison desdites trois séquences de reconnaissance est a-b-c ;
2) le vecteur recombinant M est soumis à un clivage enzymatique par l'endonucléase de restriction reconnaissant ladite séquence b, pour préparer le vecteur T ;
3) le fragment d'ADN double brin cible est préparé, et deux extrémités du fragment d'ADN double brin sont des extrémités en surplomb comportant les bases basiques libres A ;
4) le vecteur T de l'étape 2) et le fragment d'ADN double brin de l'étape 3) sont liés pour obtenir un vecteur recombinant N ;
5) le vecteur recombinant N est soumis à un clivage enzymatique par les endonucléases de restriction reconnaissant lesdites séquences a et c, pour obtenir ledit fragment O.

5. Procédé de construction d'une molécule d'ADN selon la revendication 2, 3 ou 4, dans lequel les séquences desdits a et c produisent les extrémités cohésives non liées l'une à l'autre, comportant au moins une base basique en surplomb après clivage enzymatique par des endonucléases de restriction.

6. Procédé de construction d'une molécule d'ADN selon la revendication 5, dans lequel les séquences desdits a et c produisent les extrémités cohésives non liées l'une à l'autre comportant au moins trois bases basiques en surplomb après clivage enzymatique par des endonucléases de restriction.

7. Procédé de construction d'un vecteur d'inactivation génique, qui comprend les étapes suivantes :
1) la molécule d'ADN pour expression de l'ARN en épingle à cheveux obtenu par le procédé de l'une quelconque des revendications 1 à 6 est construite par le procédé de construction selon l'une quelconque des revendications 1 à 6 ;
2) la molécule d'ADN pour expression de l'ARN en épingle à cheveux, qui est un ADN double brin, un brin étant la molécule d'ADN obtenue par le procédé selon l'une quelconque des revendications 1 à 6, un autre brin étant complémentaire de la molécule d'ADN obtenue par le procédé selon l'une quelconque des revendications 1 à 6, est construite par le procédé de construction dans lequel la molécule d'ADN obtenue par le procédé selon l'une quelconque des revendications 1 à 6 est utilisée en tant que matrice pour en synthétiser le brin complémentaire sous l'action d'une ADN polymérase, avec la capacité de déplacement de brin pour former la molécule d'ADN pour expression de l'ARN en épingle à cheveux, qui est un ADN double brin, un brin étant la molécule d'ADN obtenue par le procédé selon l'une quelconque des revendications 1 à 6, un autre brin étant complémentaire de la molécule d'ADN obtenue par le procédé selon l'une quelconque des revendications 1 à 6, ladite ADN polymérase étant en option l'ADN polymérase phi29 ;
3) la molécule d'ADN pour expression de l'ARN en épingle à cheveux, qui est un ADN double brin, un brin étant la molécule d'ADN obtenue par le procédé selon l'une quelconque des revendications 1 à 6, un autre brin étant complémentaire de la molécule d'ADN obtenue par le procédé selon l'une quelconque des revendications 1 à 6, est soumise à un clivage enzymatique par les endonucléases de restriction pour obtenir un fragment d'ADN codant pour l'ARN en épingle à cheveux, et lesdites endonucléases de restriction satisfont à l'exigence ① ou ② ci-après :
① reconnaissance de ladite séquence de reconnaissance R ;
② reconnaissance de ladite séquence de reconnaissance S ;
4) le fragment d'ADN codant pour l'ARN en épingle à cheveux obtenu dans l'étape 3) est inséré dans le site de polyclonage du vecteur d'expression de gènes pour obtenir un vecteur d'inactivation génique.

8. Utilisation du procédé de construction selon la revendication 7 pour construire une banque d'ARN en épingle à cheveux.


### Sequence List

<110> Wang Lei, Fan Yunliu
<120> DNA MOLECULE FOR EXPRESSING HAIRPIN RNA, THE CONSTRUCTING METHOD AND THE USE THEREOF
<130> P31158-WOEP
<140> 08800572.3
   <141> 2008-09-04
<150> PCT/CN2008/001576
   <151> 2008-09-04
<160> 10
<210> 1
   <211> 59
   <212> DNA
   <213> artificial sequence
<220>
   <223>
<400> 1
   gaaggcgatc gctctagaga gctggtttcc tctttaggtg agctcactag agcgatcgc 59
<210> 2
   <211> 59
   <212> DNA
   <213> artificial sequence
   <220>
   <223>
<400> 2
   tcccagggcc cactgcaggg atcggtttct cttttaggtg gatccatgca gtgggccct 59
<210> 3
   <211> 90
   <212> DNA
   <213> artificial sequence
<220>
   <223>
<400> 3
   tcccggtgct ctcgcgacag ctgttgatca atgtttttgt gtttctgagt cttcagactt 60
   tgcaatatgc agagctgtcg cgagagcacc 90
<210> 4
   <211> 49
   <212> DNA
   <213>artificial sequence
<220>
   <223>
<400> 4
   cagctgctcg agtctagagt taactttgca gctagactcg agcagctgt 49
<210> 5
   <211> 26
   <212> DNA
   <213> artificial sequence
<220>
<223>
<400> 5
   ggtctctggg atatcggtct ctgaag 26
<210> 6
   <211> 26
   <212> DNA
   <213> artificial sequence
<220>
   <223>
<400> 6
   cttcagagac cgatatccca gagacc 26
<210> 7
   <211> 67
   <212> DNA
   <213> artificial sequence
<220>
   <223>
<400> 7
   ggtctctggg atatccaagc tttaaatggt tatagccatg gaactagtgg atcccgggtc 60 tctgaag 67
<210> 8
   <211> 67
   <212> DNA
   <213> artificial sequence
<220>
   <223>
<400> 8
   cttcagagac ccgggatcca ctagttccat ggctataacc atttaaagct tggatatccc 60 agagacc 67
<210> 9
   <211> 25
   <212> DNA
   <213> artificial sequence
<220>
   <223>
<400> 9
   aagcttcggg caattgctgt gccag 25
<210> 10
   <211> 20
   <212> DNA
   <213> artificial sequence
<220>
   <223>
<400> 10
   cggcaataac atacggcgtg 20